(19) Europäisches Patentamt
European Patent Office
Office européen des brevets

(11) Veröffentlichungsnummer: **0 159 584**
**B1**

(12) **EUROPÄISCHE PATENTSCHRIFT**

(45) Veröffentlichungstag der Patentschrift: **18.01.89**

(51) Int. Cl.⁴: **C 07 D 233/56**, C 07 D 249/08, A 01 N 47/38

(21) Anmeldenummer: **85103974.3**

(22) Anmeldetag: **02.04.85**

(54) **N-(Azolylcarbamoyl)-hydroxylamine und diese enthaltende Fungizide.**

(30) Priorität: **21.04.84 DE 3415138**

(43) Veröffentlichungstag der Anmeldung:
**30.10.85 Patentblatt 85/44**

(45) Bekanntmachung des Hinweises auf die Patenterteilung:
**18.01.89 Patentblatt 89/3**

(84) Benannte Vertragsstaaten:
**AT BE CH DE FR GB IT LI NL SE**

(56) Entgegenhaltungen:
**NL-A-272 627**
**US-A-4 139 365**

(73) Patentinhaber: **BASF Aktiengesellschaft, Carl-Bosch-Strasse 38, D-6700 Ludwigshafen (DE)**

(72) Erfinder: **Rentzea, Costin, Dr., Richard-Kuhn-Strasse 1-3, D-6900 Heidelberg (DE)**
Erfinder: **Sauter, Hubert, Dr., Neckarpromenade 20, D-6800 Mannheim 1 (DE)**
Erfinder: **Karbach, Stefan, Dr., Sperlinggasse 3, D-6700 Ludwigshafen (DE)**
Erfinder: **Will, Wolfgang, Dr., Schulstrasse 46, D-6800 Mannheim 24 (DE)**
Erfinder: **Ammermann, Eberhard, Dr., Sachsenstrasse 3, D-6700 Ludwigshafen (DE)**
Erfinder: **Pommer, Ernst-Heinrich, Dr., Berliner Platz 7, D-6703 Limburgerhof (DE)**

**EP 0 159 584 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue N-(Azolylcarbamoyl)-hydroxyamine, Verfahren zu ihrer Herstellung und fungizide Mittel, die diese Verbindungen als Wirkstoff enthalten.

Es ist bekannt, N-Trichlormethylthio-tetrahydrophthalimid als Fungizid in der Landwirtschaft sowie in Obst- und Gartenbau einzusetzen (vgl. Chemical Week, June 21, 1972, Seite 46). Das bekannte Mittel ist jedoch nur vor der Infektion verwendbar und seine Wirkung genügt bei niedrigen Aufwandmengen den Anforderungen der Praxis nicht.

Es sind ferner Carbamoylpyrazole bekannt, die eine pharmakologische Wirkung haben (NL-A-272 627) sowie Carbamoylimidazole, die zur Hemmung des Längenwachstums von Pflanzen geeignet sind (US-A-4 139 365).

Es wurde nun gefunden, dass N-(Azolylcarbamoyl)-hydroxylamine der Formel

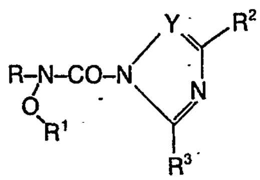

(I),

in der R einen Alkyl-, Alkenyl-, Alkoxyalkyl- oder Cycloalkylrest jeweils mit bis zu 12 C-Atomen bedeutet oder einen gegebenenfalls durch Halogen, Cyan, Nitro, Trifluormethyl, $C_1$–$C_4$-Alkyl oder $C_1$–$C_4$-Alkoxy substituierten Phenyl-, Phenyl-$C_1$–$C_4$-alkyl-Phenylalkenyl- oder Phenoxy-$C_1$–$C_6$-alkylrest bedeutet,

$R^1$ Alkyl, Alkenyl, Alkoxyalkyl, Alkylthioalkyl, Cycloalkyl oder Cycloalkylalkyl jeweils mit bis zu 12 Kohlenstoffatomen oder einen gegebenenfalls durch Halogen, $C_1$–$C_4$-Alkyl, $C_1$–$C_4$-Alkoxy, Trifluormethyl, Nitro oder Cyan substituierten Benzylrest bedeutet, und

Y CH oder N bedeutet, und

$R^2$ und $R^3$ gleich oder verschieden sind und Wasserstoff oder Alkyl mit 1 bis 5 C-Atomen bedeuten, sehr gut wirksam gegen Schadpilze sind.

Die neuen Verbindungen der Formel I enthalten chirale Zentren und werden im allgemeinen in Form von Mischungen (Racemate oder Diastereomerengemische) erhalten. Die Diastereomeren lassen sich bei einigen der erfindungsgemässen Verbindungen beispielsweise durch Säulenchromatographie trennen oder aufgrund von Löslichkeitsunterschieden in reiner Form isolieren. Aus solchen Diastereomeren kann man mit bekannten Methoden einheitliche Enantiomere erhalten. Diese werden wie ihre Mischungen von der vorliegenden Erfindung umfasst. Für die Anwendung der erfindungsgemässen Verbindungen als Fungizide sind sowohl die Diastereomeren bzw. Enantiomeren wie auch deren bei der Synthese anfallenden Gemische geeignet. Bevorzugt werden die letzteren verwendet.

Als Bedeutung für R und $R^1$ in Formel I kommen Reste wie $C_1$–$C_{12}$-Alkyl, Ethyl, n-Propyl, Isopropyl, n-Butyl, Isobutyl, sek.-Butyl, tert.-Butyl, n-Pentyl, 3-Methyl-1-butyl, n-Hexyl, 3,3-Dimethylbutyl-1, n-Heptyl, n-Octyl, 2,4,4-Trimethylpentyl, 2-Ethylhexyl, n-Decyl, n-Dodecyl, Cyclopropylmethyl, $C_5$–$C_6$-Cycloalkyl, Cyclopentyl, Cyclohexyl, 4-Methylcyclohexyl, Cyclohexylmethyl, 4-tert.-Butylcyclohexyl, $C_1$–$C_4$-Alkoxy-$C_1$–$C_4$-alkyl, 2-Methoxyethyl, 2-Ethoxyethyl, 2-Propoxyethyl, 2-Butoxyethyl, 3-Methoxypropyl, 3-Ethoxypropyl, 3-Propoxypropyl, 3-Butoxypropyl, $C_3$–$C_8$-Alkenyl, Allyl, 2-Buten-1-yl, 2-Penten-1-yl, 2-Octen-1-yl, Benzyl, 4-Methylbenzyl, alpha-Methylbenzyl, alpha-Ethylbenzyl, 2-, 3- und 4-Fluorbenzyl, 2-, 3- und 4-Chlorbenzyl, 4-Brombenzyl, 3- und 4-Trifluormethylbenzyl, 4-tert.-Butylbenzyl, p-Methoxybenzyl, 2,4-Dichlorbenzyl- oder Phenylethyl in Frage. Ferner bedeutet R Phenyl, 2-, 3- und 4-Chlorphenyl, 4-Methylphenyl, 4-Methoxyphenyl, 4-Trifluormethyl, 2,4-Dichlor- und 3,4-Dichlorphenyl, Phenoxy-ethyl, -propyl, -butyl und -hexyl, 4-Fluorphenoxy-ethyl- und -butyl, 2-, 3- und 4-Chlorphenoxy-ethyl, -propyl, -butyl und -hexyl, 2,4-, 3,4- und 2,6-Dichlorphenoxy-ethyl, -propyl, -butyl und -hexyl, 2,4,6-, 3,5,6- und 2,5,6-Trichlorphenoxy-ethyl, -propyl und -butyl, 4-Methoxyphenoxy-ethyl, -propyl und -butyl, 2-Methyl-4,6-dichlorphenoxy-ethyl, -propyl und -butyl, 2,6-Dichlor-4-bromphenoxy-ethyl, -propyl und -butyl, 4-Phenylphenoxy-ethyl, -propyl- und -butyl.

$R^2$ und $R^3$ bedeuten vorzugsweise Wasserstoff, Methyl, Ethyl, n-Propyl und Isopropyl.

Die Verbindungen der Formel I lassen sich herstellen, indem man ein Carbamoylchlorid der Formel II

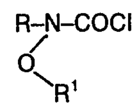

(II)

in der R und $R^1$ die oben angegebenen Bedeutungen haben,

a) mit Azolen der Formel III

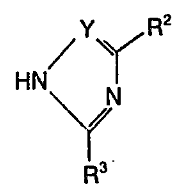

(III),

in der $R^2$, $R^3$ und Y die oben angegebenen Bedeutungen haben, oder

b) mit deren Metall-Derivaten der Formel IV

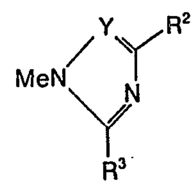

(IV),

in der $R^2$, $R^3$ und Y die oben angegebenen Bedeutungen haben und Me Lithium, Natrium, Kalium oder 1/2 Calcium bedeutet, oder

c) mit deren Silyl-Derivaten der Formel V

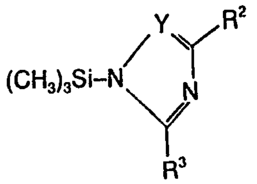 (V),

in der $R^2$, $R^3$ und Y die oben angegebenen Bedeutungen haben, umsetzt.

Die Reaktion a) erfolgt gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120 °C.

Als bevorzugte, gegenüber den Reaktionsteilnehmern, inerte Lösungs- oder Verdünnungsmittel können beispielsweise aliphatische oder aromatische, gegebenenfalls halogenierte Kohlenwasserstoffe, wie n-Pentan, Cyclohexan, Methylenchlorid, 1,1,1-Trichlorethan, Benzol, Toluol, Xylol, Chlorbenzol, aliphatische Ketone, wie Aceton, Methylethylketon, Diethylketon, Ether, wie Diethylether, Methyl-tert.-butylether, Dimethoxyethan, Tetrahydrofuran oder Dioxan, Ester, wie Essigsäureethylester, Nitrile wie Acetonitril, Amide, wie Dimethylformamid, Dimethylacetamid und N-Methylpyrrolidon oder Gemische dieser Lösungsmittel verwendet werden.

Geeignete Basen, die gegebenenfalls auch als säurebindende Mittel bei der Reaktion verwendet werden können, sind beispielsweise Alkalihydroxide wie Lithium, Natrium-, Kalium- oder Calciumhydroxid, Alkalicarbonate wie Natrium- oder Kaliumcarbonat oder Natrium- und Kaliumhydrogencarbonat, Amine wie Triethylamin, Tripropylamin, N-Methylpyrrolidin, N-Methylpiperidin, N,N-Dimethylcyclohexylamin, N,N'-Tetramethylethylendiamin, N,N-Dimethylanilin, N,N-Diethylanilin, Pyridin oder 4-Dimethylaminopyridin. Es können aber auch andere übliche Basen verwendet werden.

Als Reaktionsbeschleuniger kommen vorzugsweise Metallhalogenide wie Natriumiodid oder Kaliumiodid, quaternäre Ammoniumsalze wie Tetrabutylammonium-chlorid, -bromid oder -iodid, Benzyltriethylammonium-chlorid oder -bromid oder Kronenether wie 12-Krone-4, 15-Krone-5, 18-Krone-6 oder Dibenzo-18-krone-6 in Frage.

Die Reaktionen b) und c) erfolgen gegebenenfalls in Gegenwart eines Lösungs- oder Verdünnungsmittels bei einer Temperatur im Bereich zwischen 0 und 140 °C, vorzugsweise zwischen 0 und 100 °C. Hierfür eignen sich dieselben Lösungsmittel wie für die Verfahrensvariante a).

Die Verbindungen der Formel I lassen sich ferner herstellen, indem man eine Verbindung der Formel VI

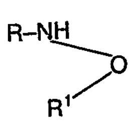 (VI),

in der R und $R^1$ die oben angegebenen Bedeutungen haben, mit einem Carbonyl-bisazol der Formel VII

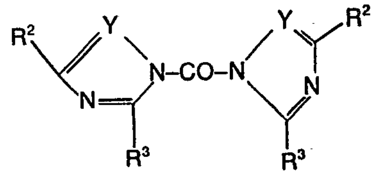 (VII),

in der $R^2$, $R^3$ und Y die oben angegebenen Bedeutungen haben, gegebenenfalls in Gegenwart eines Lösungs- oder Verdünnungsmittels, gegebenenfalls unter Zusatz eines Reaktionsbeschleunigers, umsetzt.

Hierfür eignen sich beispielsweise folgende Lösungs- oder Verdünnungsmittel: Diethylether, 1,2-Dimethoxyethan, Dipropylether, Dibutylether, Methyl-tert.-butylether, Tetrahydrofuran, Dimethoxyethan, Anisol, n-Pentan, n-Hexan, n-Heptan, n-Octan, Isooctan, Cyclohexan, Toluol, Chlorbenzol, Xylole, Acetonitril, Essigester, Dimethylformamid, N-Methylpyrrolidon, Aceton oder Methylketon.

Geeignete Reaktionsbeschleuniger sind beispielsweise 4-Dimethylaminopyridin oder 4-Pyrrolidinopyridin.

Die Ausgangsstoffe der Formel II können leicht nach bekannten Verfahren hergestellt werden, beispielsweise durch Umsetzung von Aminen der Formel VI mit Phosgen (Houben-Weyl-Müller, Methoden der organischen Chemie, Band 8, Seite 115 bis 118, Georg Thieme Verlag, Stuttgart, 1952).

Die folgenden Beispiele erläutern die Herstellung der Verbindungen der Formel I:

Beispiel 1

a) Zu einer Suspension von 48,5 g (0,5 Mol) O-Ethylhydroxylamin-chlorhydrat in 250 ml Ethyldiisopropylamin werden 30,4 g (0,1 Mol) 2,4,6-Trichlorphenoxyethylbromid zugegeben. Nach 4tägigem Rühren bei 50 °C wird das Gemisch auf 15 °C abgekühlt und abgesaugt. Das Filtrat wird im Vakuum eingeengt, schliesslich bei 60 °C und 0,1 mbar, und der Rückstand in 300 ml Ether aufgelöst und bei +3 °C mit Chlorwasserstoff begast. Man erhält 17 g O-Ethyl-N-(2,4,6-Trichlorphenoxy)-ethylhydroxylaminchlorhydrat vom Schmp. 173–175 °C.

b) Die Suspension von 15,9 g (0,05 Mol) O-Ethyl-N-(2,4,6-trichlorphenoxy)ethylhydroxylamin in 150 ml trockenem Essigester wird 8 h bei 50 °C unter gutem Rühren mit Phosgen begast. Das Gemisch wird unter vermindertem Druck eingeengt. Man erhält 17,3 g O-Ethyl-N-chlorcarbonyl-4-(2,4,6-trichlorphenoxy)-ethylhydroxylamin als farbloses Öl, das gleich weiter umgesetzt wird.

c) Zu einer Lösung von 10,3 g (0,15 Mol) Imidazol in 100 ml trockenem Tetrahydrofuran werden bei 25 °C 17,3 g (0,05 Mol) O-Ethyl-N-chlorcarbonyl-N-(2,4,6-trichlorphenoxy)-ethylhydroxylamin zugetropft. Nach 8stündigem Rühren bei 70 °C wird das Gemisch auf 20 °C abgekühlt und der gebildete Niederschlag abgesaugt. Das Filtrat wird unter vermindertem Druck eingeengt, der Rückstand in 200 ml Methylenchlorid aufgenommen, dreimal mit je 70 ml Wasser gewaschen, getrocknet und eingeengt.

Man erhält 12,5 g O-Ethyl-N-carbonylimidazol-N-(2,4,6-trichlorphenoxy)-ethylhydroxylamin als weisse Kristalle vom Schmp. 66–67 °C (Verbindung Nr. 1).

In entsprechender Weise können die in der folgenden Tabelle aufgeführten Verbindungen hergestellt werden.

| Verbindung Nr. | R | $R^1$ | Y | $R^2$ | $R^3$ | Fp [°C] IR (Film) [$cm^{-1}$] |
|---|---|---|---|---|---|---|
| 2 | $C_2H_5$ | $CH_3$ | CH | H | H | |
| 3 | $C_2H_5$ | $C_2H_5$ | CH | H | H | |
| 4 | $C_2H_5$ | $n{-}C_3H_7$ | CH | H | H | |
| 5 | $i{-}C_3H_7$ | $C_2H_5$ | CH | H | H | |
| 6 | $i{-}C_3H_7$ | $n{-}C_4H_9$ | CH | H | H | |
| 7 | $i{-}C_3H_7$ | $n{-}C_4H_9$ | N | H | H | |
| 8 | $i{-}C_3H_7$ | $n{-}C_6H_9$ | CH | H | H | |
| 9 | $i{-}C_3H_7$ | $n{-}C_6H_9$ | N | H | H | |
| 10 | $i{-}C_3H_7$ | Cyclopropylmethyl | CH | H | H | |
| 11 | $i{-}C_3H_7$ | Cyclopentyl | CH | H | H | |
| 12 | $i{-}C_3H_7$ | Cyclohexyl | CH | H | H | |
| 13 | $i{-}C_3H_7$ | $-CH_2{-}CH=CH_2$ | CH | H | H | 1692, 1460, 1409, 1369, 1314, 1285, 1203, 1178, 1100, 1063, 1006, 960, 942 |
| 14 | $i{-}C_3H_7$ | $-CH_2{-}CH=CH_2$ | N | H | H | 53–55 |
| 15 | $i{-}C_3H_7$ | $-CH_2{-}CH=CH{-}C_3H_7$ | CH | H | H | |
| 16 | $i{-}C_3H_7$ | $-(CH_2)_2OC_2H_5$ | CH | H | H | |
| 17 | $n{-}C_6H_{13}$ | $-(CH_2)_2OC_3H_7$ | CH | H | H | |
| 18 | $n{-}C_{12}H_{25}$ | $-(CH_2)_3OC_4H_5$ | CH | H | H | |
| 19 | $n{-}C_{12}H_{25}$ | $-(CH_2)_3OC_4H_5$ | N | H | H | |
| 20 | $-CH_2{-}CH=CH_2$ | $n{-}C_{12}H_{25}$ | CH | H | H | |
| 21 | $-CH_2{-}CH=CH_2$ | $n{-}C_{12}H_{25}$ | N | H | H | |
| 22 | $-CH_2{-}CH=CH{-}C_3H_7$ | $4{-}ClC_6H_4{-}CH_2{-}$ | CH | H | H | |
| 23 | Cyclohexyl | $-CH_2{-}CH=CH_2$ | CH | H | H | 28–31 |
| 24 | Cyclohexyl | $-CH_2{-}CH=CH_2$ | N | H | H | 82–84 |
| 25 | Cyclohexyl | $-CH_2{-}C_6H_5$ | CH | H | H | 74–76 |
| 26 | Cyclohexyl | $-CH_2{-}C_6H_5$ | N | H | H | 95–97 |
| 27 | Cyclohexyl | $4{-}F{-}C_6H_4{-}CH_2{-}$ | CH | H | H | |
| 28 | Cyclohexyl | $4{-}BrC_6H_4{-}CH_2{-}$ | CH | H | H | |
| 29 | Cyclohexyl | $2,4{-}Cl_2C_6H_3{-}CH_2{-}$ | CH | H | H | |
| 30 | Cyclohexyl | $3,4{-}Cl_2C_6H_3{-}CH_2{-}$ | CH | H | H | |
| 31 | 4-Methylcyclohexyl | $4{-}CF_3{-}C_6H_4{-}CH_2{-}$ | CH | H | H | |
| 32 | 4-Methylcyclohexyl | $3{-}CF_3{-}C_6H_4{-}CH_2{-}$ | CH | H | H | |
| 33 | Cycloheptyl | $-CH_2{-}C_6H_5$ | CH | H | H | |
| 34 | Cyclooctyl | $-CH_2{-}C_6H_5$ | CH | H | H | |
| 35 | Cyclododecyl | $-CH_2{-}C_6H_5$ | CH | H | H | |
| 36 | 4-tert.-Butylcyclohexyl | $-CH_2{-}C_6H_5$ | CH | H | H | |
| 37 | $C_6H_5{-}CH_2{-}$ | $-(CH_2)_2CH(CH_3)_2$ | CH | H | H | |
| 38 | $C_6H_5{-}CH_2{-}$ | $-(CH_2)_2CH(CH_3)_2$ | N | H | H | |
| 39 | $4{-}ClC_6H_4{-}CH_2{-}$ | $-CH_2CH(C_2H_5)(CH_2)_3CH_3$ | CH | H | H | |
| 40 | $3,4{-}Cl_2{-}C_6H_3{-}CH_2$ | $-(CH_2)_2CH(CH_3)CH_2C(CH_3)_3$ | CH | H | H | |
| 41 | 1-Phenyl-1-ethyl | $iso{-}C_3H_7$ | CH | H | H | 54–56 |
| 42 | 1-Phenyl-1-ethyl | $iso{-}C_3H_7$ | N | H | H | 86–88 |
| 43 | $C_6H_5{-}O{-}(CH_2)_2{-}$ | $n{-}C_6H_{13}$ | CH | H | H | |
| 44 | $C_6H_5{-}O{-}(CH_2)_2{-}$ | $n{-}C_6H_{13}$ | N | H | H | |
| 45 | $C_6H_5{-}O{-}(CH_2)_3{-}$ | $n{-}C_4H_9$ | CH | H | $CH_3$ | |
| 46 | $C_6H_5{-}O{-}(CH_2)_4{-}$ | $n{-}C_4H_9$ | CH | H | iso $C_3H_7$ | |
| 47 | $C_6H_5{-}O{-}(CH_2)_4{-}$ | $C_2H_5$ | CH | H | H | 1688, 1600, 1498, 1437, 1420, 1294, 1245, 1019, 756, 693, |
| 48 | $C_6H_5{-}O{-}(CH_2)_5{-}$ | $C_2H_5$ | CH | H | H | 1722, 1599, 1585, 1545, 1497, 1474, 1315, 1288, 1244, 1080, 755, 692 |

(Fortsetzung)

| Verbindung Nr. | R | $R^1$ | Y | $R^2$ | $R^3$ | Fp [°C] IR (Film) [$cm^{-1}$] |
|---|---|---|---|---|---|---|
| 49 | $2-ClC_6H_4-O(CH_2)_4-$ | $n-C_4H_9$ | CH | H | H | |
| 50 | $3-ClC_6H_4-O(CH_2)_4-$ | $-CH_2-C_6H_5$ | CH | H | H | |
| 51 | $4-ClC_6H_4-O-(CH_2)_2-$ | $n-C_6H_{13}$ | CH | H | H | |
| 52 | $2-FC_6H_4-O-(CH_2)_4-$ | $C_2H_5-$ | CH | H | H | 1688, 1507, 1472, 1457, 1436, 1419, 1311, 1281, 1259, 1205, 1109, 1063, 1019, 749 |
| 53 | $3,4-Cl_2C_6H_3-O-(CH_2)_5-$ | $C_2H_5-$ | CH | H | H | 69–71 |
| 54 | $3,4-Cl_2C_6H_3-O-(CH_2)_2-$ | $C_2H_5-$ | N | H | H | 108–111 |
| 55 | $2,6-Cl_2C_6H_3-O-(CH_2)_2-$ | $C_2H_5-$ | N | H | H | |
| 56 | $4-CH_3-C_6H_4-O-(CH_2)_4-$ | $-CH_2-CH=CH_2$ | CH | H | H | |
| 57 | $4-tert.-C_4H_9-C_6H_4-O-(CH_2)_4-$ | $n-C_6H_{13}$ | CH | H | H | |
| 58 | $4-CH_3O-C_6H_4-O-(CH_2)_4-$ | $n-C_4H_9$ | CH | H | H | |
| 59 | $4-C_2H_5O-C_6H_4-O-(CH_2)_4-$ | $n-C_4H_9$ | CH | H | H | |
| 60 | $4-CF_3C_6H_4-O-(CH_2)_2-$ | $n-C_6H_{13}$ | CH | H | H | |
| 61 | $4-CNC_6H_4-O-(CH_2)_6-$ | $n-C_5H_{11}$ | CH | H | H | |
| 62 | $2,4,6-Cl_3C_6H_2-O-(CH_2)_3-$ | $C_2H_5$ | CH | H | H | 1689, 1551, 1446, 1418, 1385, 1298, 1258, 1246, 1229, 1062, 1022, 1006, 856 |
| 63 | $2,4,6-Cl_3C_6H_2-O-(CH_2)_3-$ | $-CH_2-CH=CH_2$ | CH | H | H | 1690, 1552, 1447, 1419, 1384, 1297, 1258, 1248, 1228, 1062, 1007, 937, 857, 799 |
| 64 | $2,4,6-Cl_3C_6H_2-O-(CH_2)_4-$ | $C_2H_5$ | CH | H | H | 1688, 1551, 1439, 1419, 1386, 1279, 1257, 1216, 1064, 1019, 856, 814, 799, 733 |
| 65 | $2,6-Cl_2-4Br-C_6H_2-O(CH_2)_2-$ | $C_2H_5$ | CH | H | H | 1690, 1463, 1448, 1434, 1380, 1253, 1240, 1062, 1021, 1005, 807, 750, 733 |
| 66 | $2-CH_3$, $4,6-Cl_2C_6H_2-O(CH_2)_2-$ | $C_2H_5$ | CH | H | H | 1692, 1463, 1434, 1389, 1281, 1261, 1212, 1177, 1062, 1043, 1021, 1006, 854, 759 |
| 67 | $4-C_6H_5-2ClC_6H_3-O(CH_2)_2-$ | $C_2H_5$ | CH | H | H | 70–72 |

Die neuen Wirkstoffe zeigen eine starke fungitoxische Wirksamkeit gegen phytopathogene Pilze. Sie dienen insbesondere zur Verhütung und Heilung von Pflanzenkrankheiten, die durch Mikroorganismen verursacht werden, wie z.B. Phytophthora infestans, Botrytis cinerea, Plasmopara viticola, Monilia fructigena, Alternaria solani, Sclerotinia sclerotiorum, Pyricularia oryzae, Pellicularia filamentosa, Sclerotinia cinerea.

Die neuen Wirkstoffe sind ausserdem wirksam gegen Candida albicans und Trichophyton mentagrophytes.

Die Wirkstoffe können gleichzeitig das Wachstum von zwei oder mehr der genannten Pilze unterdrücken und besitzen eine hohe Pflanzenverträglichkeit. Ein Teil der Wirkstoffe zeigt kurative Eigenschaften. d.h. die Anwendung der Mittel kann noch nach erfolgter Infektion der Pflanzen durch die Krankheitserreger vorgenommen werden, um einen sicheren Bekämpfungserfolg zu erzielen.

Die fungiziden Mittel enthalten 0,1 bis 95% (Gewichtsprozent) Wirkstoff, vorzugsweise 0,5 bis 90%. Die Aufwandmengen liegen je nach Art des gewünschten Effektes zwischen 0,1 bis 5 kg Wirkstoff je ha.

Die Wirkstoffe können auch zusammen mit anderen Wirkstoffen, z.B. Herbiziden, Insektiziden, Wachstumsregulatoren und anderen Fungiziden, oder auch mit Düngemitteln vermischt ausgebracht werden. In vielen Fällen erhält man bei der Mischung mit Fungiziden auch eine Vergrösserung des fungiziden Wirkungsspektrums; bei einer Anzahl dieser Fungizidmischungen treten auch synergistische Effekte auf, d. h. die fungizide Wirksamkeit des Kombinationsproduktes ist grösser als die der addierten Wirksamkeiten der Einzelkomponenten.

Fungizide, die mit den neuen Verbindungen kombiniert werden können, sind beispielsweise:
Schwefel,
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethyl-acrylat
2-sec.-Butyl-4,6-dinitrophenyl-isopropylcarbonat
5-Nitro-isophthalsäure-di-isopropylester,

heterocyclische Strukturen, wie
2-Heptadecyl-2-imidazolin-acetat
2,4-Dichlor-6-(o-chloranilino)-s-triazin
O,O-Diethyl-phthalimidophosphonothioat
5-Amino-1-(bis-(dimethylamino)-phosphinyl)-3-phenyl-1,2,4-triazol)
2,3-Dicyano-1,4-dithiaanthrachinon
2-Thio-1,3-dithio-(4,5,6)-chinoxalin
1-(Butylcarbamoyl)-2-benzimidazol-carbaminsäuremethylester
2-Methoxycarbonylamino-benzimidazol
2-(Furyl-(2)-benzimidazol
2-(Thiazolyl-(4)-benzimidazol
N-(1,1,2,2-Tetrachlorethylthio)-tetrahydrophthalimid
N-Trichlormethylthio-tetrahydrophthalimid
N-Trichlormethylthio-phthalimid
N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Jod-benzoesäure-anilid
N-Formyl-N-morpholin-2,2,2-trichlorethylacetal
Piperazin-1,4-diylbis-(1-(2,2,2-trichlorethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1[2-(2,4-Dichlorphenyl)-4-n-propyl-1,3-dioxolan-2-yl-ethyl]-1-H-1,2,4-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
alpha-(2-Chlorphenyl-alpha-(4-chlorphenyl)-5-pyrimidin-methanol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol

sowie verschiedene Fungizide, wie
Dodecylguanidinacetat
3-(3-(3,5-Dimethyl-2-oxycyclohexyl)-2-hydroxyethyl)-glutarimid
Hexachlorbenzol
DL-Methyl-N-(2,6-dimethyl-phenyl)-N-furoyl(2)-alaninat
DL-N-(2,6-Dimethyl-phenyl)-N-(2'-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl)-5-methyl-5-methoxymethyl-1,3-oxazolidin-2,4-dion
3-(3,5-Dichlorphenyl)-1-isopropylcarbamoyl-hydantoin
N-(3,5-Dichlorphenyl)1,2-dimethylcyclopropan-1,2-dicarbonsäureimid
2-Cyano-N-(ethylaminocarbonyl)-2-methoximino)-acetamid
1-(2-(2,4-Dichlorphenyl)-pentyl)-1H-1,2,4-triazol
2,3-Difluor-alpha-(1H-1,2,4-triazolyl-1-methyl)-benzhydrylalkohol.

Die neuen Wirkstoffe werden beispielsweise in Form von direkt versprühbaren Lösungen, Suspensionen, Emulsionen, auch in Form von hochprozentigen wässrigen, öligen oder sonstigen Dispersionen, Pasten, Stäubemitteln, Streumitteln, Granulaten durch Versprühen, Verstäuben, Verstreuen, Verstreichen oder Giessen ausgebracht. Die Anwendungsformen richten sich ganz nach den Verwendungszwecken; sie sollten in der Regel möglichst eine feine Verteilung der neuen Wirkstoffe gewährleisten.

Zur Herstellung von direkt oder nach Emulgieren in Wasser verwendbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische oder aromatische Kohlenwasserstoffe, zum Beispiel Benzol, Toluol, Xylol, Paraffin, Tetrahydronaphthalin, alkylierte Naphthaline oder deren Derivate z.B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformamid, Dimethylsulfoxid, N-Methylpyrrolidon, Wasser usw. in Betracht.

Wässrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löss, Ton, Dolomit, Diatomeenerde, Calcium- und Magnesiumsulfat, Magnesiumoxid, gemahlene Kunststoffe, Düngemittel wie z.B. Ammoniumsulfat, Ammoniumphosphat, Ammoniumnitrat, Harnstoffe und pflanzliche Produkte, wie Getreidemehle, Baumrinden-, Holz- und Nussschalenmehl, Cellulosepulver und andere feste Trägerstoffe.

Beispiele für solche Pflanzenschutzmittel-Zubereitungen sind:

I. Man vermischt 90 Gewichtsteile der Verbindung 1 mit 100 Gewichtsteilen N-Methylpyrrolidon und erhält eine Lösung, die zur Anwendung in Form kleinster Tropfen geeignet ist.

II. 20 Gewichtsteile der Verbindung 52 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgiessen und feines Verteilen der Lösung in Wasser erhält man eine wässrige Dispersion.

III. 20 Gewichtsteile der Verbindung 65 werden in einer Mischung gelöst, die aus 30 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingiessen und Verrühren der Lösung in Wasser erhält man eine wässrige Dispersion.

IV. 20 Gewichtsteile der Verbindung 64 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingiessen und Verrühren der Lösung in Wasser, erhält man eine wässrige Dispersion.

V. 20 Gewichtsteile der Verbindung 62 werden mit 3 Gewichtsteilen des Natriumsalzes der Diisobutylnaphthalin-alpha-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in Wasser erhält man eine Spritzbrühe.

VI. 5 Gewichtsteile der Verbindung 1 werden mit 95 Gewichtsteilen feinteiligem Kaolin innig vermischt. Man erhält auf diese Weise ein Stäubemittel, das 5 Gew.-% des Wirkstoffs erhält.

VII. 30 Gewichtsteile der Verbindung 52 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man

erhält auf diese Weise eine Aufbereitung des Wirkstoffs mit guter Haftfähigkeit.

VIII. 40 Gewichtsteile der Verbindung 65 werden mit 30 Teilen Natriumsalz eines Phenolsulfonsäure-Harnstoff-Formaldehyd-Kondensates, 2 Teilen Kieselgel und 48 Teilen Wasser innig vermischt. Man erhält eine stabile wässrige Dispersion.

IX. 20 Teile der Verbindung 62 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoffformaldehyd-Kondensates und 68 Teilen eines paraffinischen Mineralöls innig vermischt. Man erhält eine stabile ölige Dispersion.

Die folgenden Versuche belegen die biologische Wirkung der neuen Verbindungen. Vergleichsmittel ist der bekannte, insbesondere zur Bekämpfung von Botrytis geeignete Wirkstoff N-Trichlormethylthio-tetra-hydrophthalimid (A).

Versuch 1

Wirksamkeit gegen Botrytis cinerea an Paprika

Paprikasämlinge der Sorte «Neusiedler Ideal Elite» werden, nachdem sich 4 bis 5 Blätter gut entwickelt haben, mit wässrigen Suspensionen, die 80% Wirkstoff und 20% Emulgiermittel in der Trockensubstanz enthalten, tropfnass gespritzt. Nach dem Antrocknen des Spritzbelages werden die Pflanzen mit einer Konidienaufschwemmung des Pilzes Botrytis cinerea besprüht und bei 22 bis 24 °C in eine Kammer mit hoher Luftfeuchtigkeit gestellt. Nach 5 Tagen hat sich die Krankheit auf den unbehandelten Kontrollpflanzen so stark entwickelt, dass die entstandenen Blattnekrosen den überwiegenden Teil der Blätter bedecken.

Das Ergebnis des Versuches zeigt, dass beispielsweise bei der Anwendung als 0,05%ige Wirkstoffbrühe die Verbindungen 1, 52 und 66 eine bessere fungizide Wirkung zeigen (beispielsweise 97%) als der bekannte Wirkstoff A (70%).

Versuch 2

Wirksamkeit gegen Pyricularia oryzae (protektiv)

Blätter von in Töpfen gewachsenen Reiskeimlingen der Sorte «Bahia» werden mit wässrigen Emulsionen, die 80% Wirkstoff und 20% Emulgiermittel in der Trockensubstanz enthalten, tropfnass besprüht und 24 Stunden später mit einer wässrigen Sporensuspension von Pyricularia oryzae inoculiert. Anschliessend werden die Versuchspflanzen in Klimakammern bei 22 bis 24 °C und 95 bis 99% relativer Luftfeuchtigkeit aufgestellt. Nach 6 Tagen wird das Ausmass des Krankheitsbefalls ermittelt.

Das Ergebnis des Versuches zeigt, dass beispielsweise bei der Anwendung als 0,05%ige Wirkstoffbrühe die Verbindungen 63 und 65 eine gute fungizide Wirksamkeit (beispielsweise 90%) zeigen.

## Patentansprüche

1. N-(Azolylcarbamoyl)-hydroxylamin der Formel

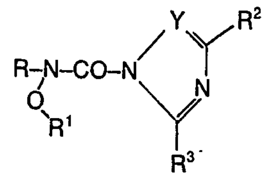

(I),

in der R einen Alkyl-, Alkenyl-, Alkoxyalkyl- oder Cycloalkylrest jeweils mit bis zu 12 C-Atomen bedeutet oder einen gegebenenfalls durch Halogen, Cyan, Nitro, Trifluormethyl, $C_1$–$C_4$-Alkyl oder $C_1$–$C_4$-Alkoxy substituierten Phenyl-, Phenylalkyl-, Phenylalkenyl- oder Phenoxyalkylrest bedeutet,

$R^1$ Alkyl, Alkenyl, Alkoxyalkyl, Alkylthioalkyl, Cycloalkyl oder Cycloalkylalkyl jeweils mit bis zu 12 Kohlenstoffatomen oder einen gegebenenfalls durch Halogen, $C_1$–$C_4$-Alkyl, $C_1$–$C_4$-Alkoxy, Trifluormethyl, Nitro oder Cyan substituierten Benzylrest bedeutet, und

Y CH oder N bedeutet, und

$R^2$ und $R^3$ gleich oder verschieden sind und Wasserstoff oder Alkyl mit 1 bis 5 C-Atomen bedeuten.

2. Fungizides Mittel, enthaltend einen inerten Zusatzstoff und ein N-(Azolylcarbamoyl)-hydroxylamin der Formel

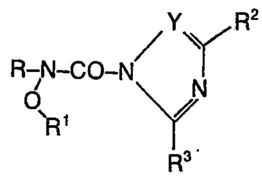

(I),

in der R einen Alkyl-, Alkenyl-, Alkoxyalkyl- oder Cycloalkylrest jeweils mit bis zu 12 C-Atomen oder einen gegebenenfalls durch Halogen, Cyan, Nitro, Trifluormethyl, $C_1$–$C_4$-Alkyl oder $C_1$–$C_4$-Alkoxy substituierten Phenyl-, Phenylalkyl-, Phenylalkenyl- oder Phenoxyalkylrest bedeutet,

$R^1$ Alkyl, Alkenyl, Alkoxyalkyl, Alkylthioalkyl, Cycloalkyl oder Cycloalkylalkyl jeweils mit bis zu 12 Kohlenstoffatomen oder einen gegebenenfalls durch Halogen, $C_1$–$C_4$-Alkyl, $C_1$–$C_4$-Alkoxy, Trifluormethyl, Nitro oder Cyan substituierten Benzylrest bedeutet, und

Y CH oder N bedeutet, und

$R^2$ und $R^3$ gleich oder verschieden sind und Wasserstoff oder Alkyl mit 1 bis 5 C-Atomen bedeuten.

3. Verfahren zur Herstellung eines fungiziden Mittels, dadurch gekennzeichnet, dass man einen inerten Zusatzstoff vermischt mit einer Verbindung der Formel I gemäss Anspruch 1.

4. Verfahren zur Bekämpfung von Pilzen, dadurch gekennzeichnet, dass man die Pilze oder die von Pilzbefall bedrohten Materialien, Flächen, Pflanzen oder Saatgüter behandelt mit einer fungizid wirksamen Menge eines N-(Azolylcarbamoyl)-hydroxylamins der Formel

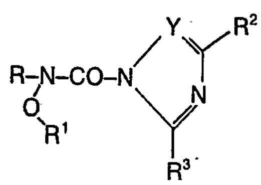

(I),

in der R einen Alkyl-, Alkenyl-, Alkoxyalkyl- oder Cycloalkylrest jeweils mit bis zu 12 C-Atomen oder einen gegebenenfalls durch Halogen, Cyan, Nitro, Trifluormethyl, $C_1$–$C_4$-Alkyl oder $C_1$–$C_4$-Alkoxy substituierten Phenyl-, Phenylalkyl-, Phenylalkenyl- oder Phenoxyalkylrest bedeutet,

$R^1$ Alkyl, Alkenyl, Alkoxyalkyl, Alkylthioalkyl, Cycloalkyl oder Cycloalkylalkyl jeweils mit bis zu 12 Kohlenstoffatomen oder einen gegebenenfalls durch Halogen, $C_1$–$C_4$-Alkyl, $C_1$–$C_4$-Alkoxy, Trifluormethyl, Nitro oder Cyan substituierten Benzylrest bedeutet, und

Y CH oder N bedeutet, und

$R^2$ und $R^3$ gleich oder verschieden sind und Wasserstoff oder Alkyl mit 1 bis 5 C-Atomen bedeuten.

5. Verfahren zur Herstellung von N-(Azolylcarbamoyl)-hydroxylaminen der Formel I gemäss Anspruch 1, dadurch gekennzeichnet, dass man eine Verbindung der Formel

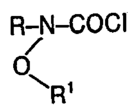 (II)

in der R und $R^1$ die im Anspruch 1 angegebenen Bedeutungen haben,

a) mit Azolen der Formel

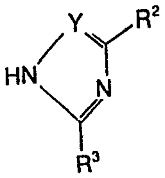 (III),

in der $R^2$, $R^3$ und Y die im Anspruch 1 angegebenen Bedeutungen haben, oder

b) mit deren Metall-Derivaten der Formel

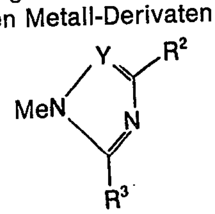 (IV),

in der $R^2$, $R^3$ und Y die im Anspruch 1 angegebenen Bedeutungen haben und Me Lithium, Natrium, Kalium oder 1/2 Calium bedeutet, oder

c) mit deren Silyl-Derivaten der Formel

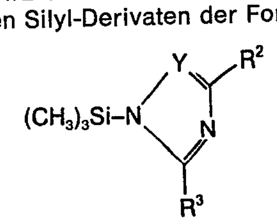 (V),

in der $R^2$, $R^3$ und Y die im Anspruch 1 angegebenen Bedeutungen haben, umsetzt.

6. Verfahren zur Herstellung von N-(Azolylcarbamoyl)-hydroxylaminen der Formel I gemäss Anspruch 1, dadurch gekennzeichnet, dass man eine Verbindung der Formel

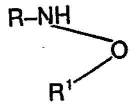 (VI),

in der R und $R^1$ die im Anspruch 1 angegebenen Bedeutungen haben, mit einem Carbonyl-bisazol der Formel

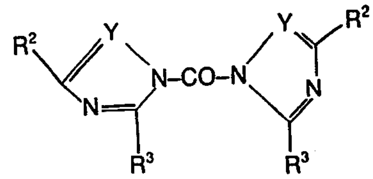 (VII),

in der $R^2$, $R^3$ und Y die im Anspruch 1 angegebenen Bedeutungen haben, umsetzt.

7. N-(Azolylcarbamoyl)-hydroxylamin der Formel I gemäss Anspruch 1, dadurch gekennzeichnet, dass R und $R^1$ Ethyl, n-Propyl, Isopropyl, n-Butyl, Isobutyl, sek.-Butyl, tert.-Butyl, n-Pentyl, 3-Methyl-1-butyl, n-Hexyl, 3,3-Dimethylbutyl-1, n-Heptyl, n-Octyl, 2,4,4-Trimethylpentyl, 2-Ethylhexyl, n-Decyl, n-Dodecyl, Cyclopropylmethyl, Cyclopentyl, Cyclohexyl, 4-Methylcyclohexyl, Cyclohexylmethyl, 4-tert.-Butylcyclohexyl, 2-Methoxyethyl, 2-Ethoxyethyl, 2-Propoxyethyl, 2-Butoxyethyl, 3-Methoxypropyl, 3-Ethoxypropyl, 3-Propoxypropyl, 3-Butoxypropyl, Allyl, 2-Buten-1-yl, 2-Penten-1-yl, 2-Octen-1-yl, Benzyl, 4-Methylbenzyl, alpha-Methylbenzyl, alpha-Ethylbenzyl, 2-, 3- und 4-Fluorbenzyl, 2-, 3- und 4-Chlorbenzyl, 4-Brombenzyl, 3- und 4-Trifluormethylbenzyl, 4-tert.-Butylbenzyl, p-Methoxybenzyl, 2,4-Dichlorbenzyl oder Phenylethyl bedeuten und R zusätzlich Phenyl, 2-, 3- und 4-Chlorphenyl, 4-Methylphenyl, 4-Methoxyphenyl, 4-Trifluormethyl, 2,4-Dichlor- und 3,4-Dichlorphenyl, Phenoxy-ethyl, -propyl, -butyl und -hexyl, 4-Fluorphenoxy-ethyl und -butyl, 2-, 3- und 4-Chlorphenoxy-ethyl, -propyl, -butyl und hexyl, 2,4-, 3,4- und 2,6-Dichlorphenoxy-ethyl, -propyl, -butyl und -hexyl, 2,4,6-, 3,5,6- und 2,5,6-Trichlorphenoxy-ethyl, -propyl und -butyl, 4-Methoxyphenoxy-ethyl, -propyl und -butyl, 2-Methyl-4,6-dichlorphenoxy-ethyl, -propyl und -butyl, 2,6-Dichlor-4-bromphenoxy-ethyl, -propyl und -butyl, 4-Phenylphenoxy-ethyl, -propyl und -butyl bedeutet, und $R^2$ und $R^3$ Wasserstoff, Methyl oder Isopropyl bedeuten.

8. N-(Azolylcarbamoyl)-hydroxylamin der Formel I gemäss Anspruch 1, dadurch gekennzeichnet, dass R 4-Fluorphenoxy-butyl, 2,4,6-Trichlorphenoxy-propyl, 2,6-Dichlor-4-bromphenoxy-ethyl, 4,6-Dichlor-2-methylphenoxy-ethyl, $R^1$ Ethyl oder Allyl, Y CH und $R^2$ und $R^3$ Wasserstoff bedeuten.

## Claims

1. An N-(azolylcarbamoyl)-hydroxylamine of the formula

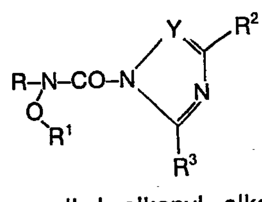 (I)

where R is an alkyl, alkenyl, alkoxyalkyl or cycloalkyl radical, each of not more than 12 carbon atoms, or is a phenyl, phenylalkyl, phenylalkenyl

or phenoxyalkyl radical which is unsubstituted or substituted by halogen, cyano, nitro, trifluoromethyl, $C_1$–$C_4$-alkyl or $C_1$–$C_4$-alkoxy,

$R^1$ is alkyl, alkenyl, alkoxyalkyl, alkylthioalkyl, cycloalkyl or cycloalkylalkyl, each of not more than 12 carbon atoms, or is benzyl which is unsubstituted or substituted by halogen, $C_1$–$C_4$-alkyl, $C_1$–$C_4$-alkoxy, trifluoromethyl, nitro or cyano,

Y is CH or N and

$R^2$ and $R^3$ are identical or different and are each hydrogen or alkyl of 1 to 5 carbon atoms.

2. A fungicide containing an inert additive and an N-(azolylcarbamoyl)-hydroxylamine of the formula

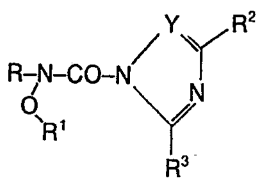

(I)

where R is an alkyl, alkenyl, alkoxyalkyl or cycloalkyl radical, each of not more than 12 carbon atoms, or is a phenyl, phenylalkyl, phenylalkenyl or phenoxyalkyl radical which is unsubstituted or substituted by halogen, cyano, nitro, trifluoromethyl, $C_1$–$C_4$-alkyl or $C_1$–$C_4$-alkoxy,

$R^1$ is alkyl, alkenyl, alkoxyalkyl, alkylthioalkyl, cycloalkyl or cycloalkylalkyl, each of not more than 12 carbon atoms, or is benzyl which is unsubstituted or substituted by halogen, $C_1$–$C_4$-alkyl, $C_1$–$C_4$-alkoxy, trifluoromethyl, nitro or cyano,

Y is CH or N and

$R^2$ and $R^3$ are identical or different and are each hydrogen or alkyl of 1 to 5 carbon atoms.

3. A process for the preparation of a fungicide, wherein an inert additive is mixed with a compound of the formula I as claimed in claim 1.

4. A method for controlling fungi, wherein the fungi or the materials, areas, plants or seed threatened by a fungal attack are treated with a fungicidally effective amount of an N-(azolylcarbamoyl)-hydroxylamine of the formula

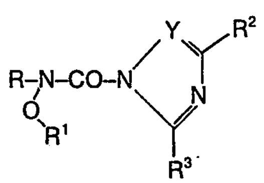

(I).

where R is an alkyl, alkenyl, alkoxyalkyl or cycloalkyl radical, each of not more than 12 carbon atoms, or is a phenyl, phenylalkyl, phenylalkenyl or phenoxyalkyl radical which is unsubstituted or substituted by halogen, cyano, nitro, trifluoromethyl, $C_1$–$C_4$-alkyl or $C_1$–$C_4$-alkoxy,

$R^1$ is alkyl, alkenyl, alkoxyalkyl, alkylthioalkyl, cycloalkyl or cycloalkylalkyl, each of not more than 12 carbon atoms, or is benzyl which is unsubstituted or substituted by halogen, $C_1$–$C_4$-alkyl, $C_1$–$C_4$-alkoxy, trifluoromethyl, nitro or cyano,

Y is CH or N and

$R^2$ and $R^3$ are identical or different and are each hydrogen or alkyl of 1 to 5 carbon atoms.

5. A process for the preparation of an N-(azolylcarbamoyl)-hydroxylamine of the formula I as claimed in claim 1, wherein a compound of the formula

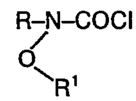

(II)

where R and $R^1$ have the meanings stated in claim 1, is reacted

a) with an azole of the formula

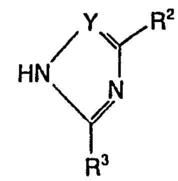

(III)

where $R^2$, $R^3$ and Y have the meanings stated in claim 1, or

b) with one of its metal derivatives of the formula

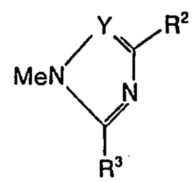

(IV)

where $R^2$, $R^3$ and Y have the meanings stated in claim 1 and Me is lithium, sodium, potassium or calcium, or

c) with one of its silyl derivatives of the formula

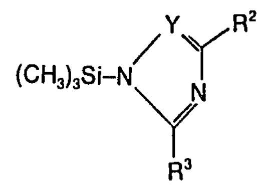

(V)

where $R^2$, $R^3$ and Y have the meanings stated in claim 1.

6. A process for the preparation of an N-(azolylcarbamoyl)-hydroxylamine of the formula I as claimed in claim 1, wherein a compound of the formula

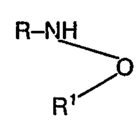

(VI),

where R and $R^1$ have the meanings stated in claim 1, is reacted with a carbonylbisazole of the formula

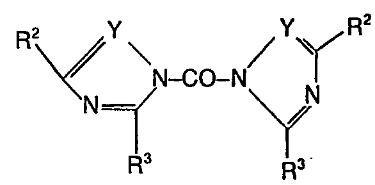

(VII)

where $R^2$, $R^3$ and Y have the meanings stated in claim 1.

7. An N-(azolylcarbamoyl)-hydroxylamine of the formula I as claimed in claim 1, wherein R and $R^1$ are each ethyl, n-propyl, isopropyl, n-butyl, isobutyl, sec-butyl, tert-butyl, n-pentyl, 3-methyl-1-butyl, n-hexyl, 3,3-di-methylbut-1-yl, n-heptyl, n-octyl, 2,4,4-trimethylpentyl, 2-ethylhexyl, n-decyl, n-dodecyl, cyclopropylmethyl, cyclopentyl, cyclohexyl, 4-methylcyclohexyl, cyclohexylmethyl, 4-tert-butylcyclohexyl, 2-methoxyethyl, 2-ethoxyethyl, 2-propoxyethyl, 2-butoxyethyl, 3-methoxypropyl, 3-ethoxypropyl, 3-propoxypropyl, 3-butoxypropyl, allyl, 2-buten-1-yl, 2-penten-1-yl, 2-octen-1-yl, benzyl, 4-methylbenzyl, alpha-methylbenzyl, alpha-ethylbenzyl, 2-, 3- and 4-fluorobenzyl, 2-, 3- and 4-chlorobenzyl, 4-bromobenzyl, 3- and 4-trifluoromethylbenzyl, 4-tert-butylbenzyl, p-methoxybenzyl, 2,4-dichlorobenzyl or phenylethyl, and R is additionally phenyl, 2-, 3- and 4-chlorophenyl, 4-methylphenyl, 4-methoxyphenyl, 4-trifluoromethyl, 2,4-dichlor- or 3,4-dichlorophenyl, phenoxy-ethyl, -propyl, -butyl or -hexyl, 4-fluorophenoxy-ethyl or -butyl, 2-, 3- or 4-chlorophenoxy-ethyl, -propyl, -butyl or -hexyl, 2,4-, 3,4- or 2,6-dichlorophenoxy-ethyl, -propyl, -butyl or -hexyl, 2,4,6-, 3,5,6- or 2,5,6-trichlorophenoxy-ethyl, -propyl or -butyl, 4-methoxyphenoxyethyl, -propyl or -butyl, 2-methyl-4,6-dichlorophenoxy-ethyl, -propyl or -butyl, 2,6-dichloro-4-bromophenoxyethyl, -propyl or -butyl, 4-phenylphenoxy-ethyl, -propyl or -butyl, and $R^2$ and $R^3$ are each hydrogen, methyl or isopropyl.

8. An N-(azolylcarbamoyl)-hydroxylamine of the formula I as claimed in claim 1, wherein R is 4-fluorophenoxybutyl, 2,4,6-trichlorophenoxypropyl, 2,6-dichloro-4-bromophenoxyethyl, 4,6-dichloro-2-methylphenoxymethyl, $R^1$ is ethyl or allyl, Y is CH and $R^2$ and $R^3$ are each hydrogen.

**Revendications**

1. N-(Azolylcarbamoyl)-hydroxylamine de formule

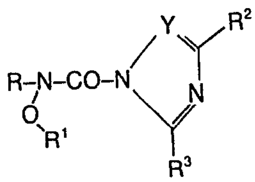 (I)

dans laquelle

R représente un reste alkyle, alcényle, alcoxyalkyle ou cycloalkyle, chacun ayant jusqu'à 12 atomes C, ou représente un reste phényle, phénylalkyle, phénylacényle ou phénoxyalkyle, éventuellement substitué par halogène, cyano, nitro, trifluorométhyle, alkyle en $C_1$–$C_4$ ou alcoxy en $C_1$–$C_4$

$R^1$ représente alkyle, alcényle, alcoxyalkyle, alkylthioalkyle, cycloalkyle ou cycloalkylalkyle, chacun ayant jusqu'à 12 atomes de carbone ou un reste benzyle, éventuellement substitué par halogène, alkyle en $C_1$–$C_4$ ou alcoxy en $C_1$–$C_4$ trifluorométhyle, nitro ou cyano, et

Y représente CH ou N et

$R^2$ et $R^3$ sont identiques ou différents et représentent hydrogène ou alkyle à 1 à 5 atomes C.

2. Fongicide, contenant un additif inerte et un N-(Azolylcarbamoyl)-hydroxylamine de formule

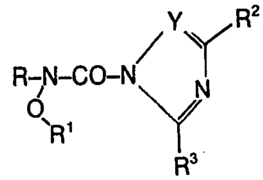 (I)

dans laquelle

R représente un reste alkyle, alcényle, alcoxyalkyle ou cycloalkyle, chacun ayant jusqu'à 12 atomes C, ou représente un reste phényle, phénylalkyle, phénylacényle ou phénoxyalkyle, éventuellement substitué par halogène, cyano, nitro, trifluorométhyle, alkyle en $C_1$–$C_4$ ou alcoxy en $C_1$–$C_4$

$R^1$ représente alkyle, alcényle, alcoxyalkyle, alkylthioalkyle, cycloalkyle ou cycloalkylalkyle, chacun ayant jusqu'à 12 atomes de carbone ou un reste benzyle, éventuellement substitué par halogène, alkyle en $C_1$–$C_4$ ou alcoxy en $C_1$–$C_4$ trifluorométhyle, nitro ou cyano, et

Y représente CH ou N et

$R^2$ et $R^3$ sont identiques ou différents et représentent hydrogène ou alkyle à 1 à 5 atomes C.

3. Procédé de préparation d'un fongicide caractérisé par le fait qu'on mélange un additif inerte avec un composé de formule I selon la revendication 1.

4. Procédé de lutte contre les champignons, caractérisé par le fait qu'on traite les champignons ou les matériaux, surfaces, plantes ou semences menacés par une attaque par les champignons, avec une quantité efficace du point de vue fongicide d'un N-(Azolylcarbamoyl)-hydroxylamine de formule

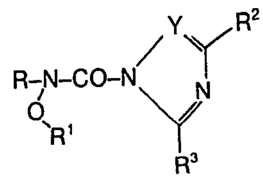 (I)

dans laquelle

R représente un reste alkyle, alcényle, alcoxyalkyle ou cycloalkyle, chacun ayant jusqu'à 12 atomes C, ou représente un reste phényle, phénylalkyle, phénylacényle ou phénoxyalkyle, éventuellement substitué par halogène, cyano, nitro, trifluorométhyle, alkyle en $C_1$–$C_4$ ou alcoxy en $C_1$–$C_4$,

$R^1$ représente alkyle, alcényle, alcoxyalkyle, alkylthioalkyle, cycloalkyle ou cycloalkylalkyle, chacun ayant jusqu'à 12 atomes de carbone ou un reste benzyle, éventuellement substitué par halogène, alkyle en $C_1$–$C_4$ ou alcoxy en $C_1$–$C_4$ trifluorométhyle, nitro ou cyano, et

Y représente CH ou N et

$R^2$ et $R^3$ sont identiques ou différents et représentent hydrogène ou alkyle à 1 à 5 atomes C.

5. Procédé de préparation de N-(azolylcarbamoyl)-hydroxylamines de formule I selon la revendication 1, caractérisé par le fait qu'on fait réagir un composé de formule

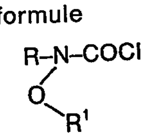 (II)

dans laquelle R et $R^1$ ont les significations indiquées dans la revendication 1

a) avec des azoles de formule

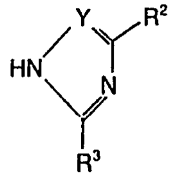

(III)

dans laquelle $R^2$, $R^3$ et Y ont les significations données dans la revendication 1, ou

b) avec leurs dérivés métalliques de formule

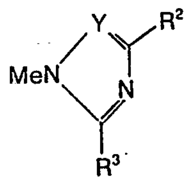

(IV)

dans laquelle $R^2$, $R^3$ et Y ont les significations données dans la revendication 1 et Me représente lithium, sodium, potassium ou ½ calcium

c) avec leurs dérivés silyliques de formule

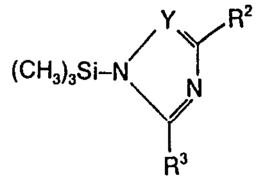

(V)

dans laquelle $R^2$, $R^3$ et Y ont les significations données dans la revendication 1.

6. Procédé de préparation de N-(azolylcarbamoyl)-hydroxyamines de formule I selon la revendication 1, caractérisé par le fait qu'on fait réagir un composé de formule

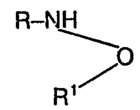

(VI)

dans laquelle R et $R^1$ ont les significations indiquées dans la revendication 1, avec un carbonylbisazole de formule

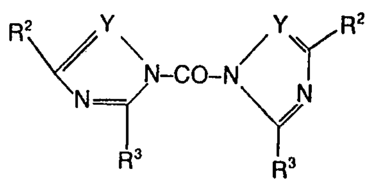

(VII)

dans lesquelles $R^2$, $R^3$ et Y ont les significations indiquées dans la revendication 1.

7. N-(azolylcarbamoyl)-hydroxylamine de formule I selon la revendication 1, caractérisé par le fait que R et $R^1$ représentent éthyle, n-propyle, isopropyle, n-butyle, isobutyle, sec.-butyle, tert.-butyle, N-pentyle, 3-méthyl-1-butyle, n-héxyle, 3,3-dimethylbutyl-1, n-heptyle, n-octyle, 2,4,4-trimethylpentyle, 2-ethylhexyle, n-decyle, n-dodecyle, cyclopropylmethyle, cyclopentyle, cyclohexyle, 4-methylcyclohexyle, cyclohexylmethyle, 4-tert.-butylcyclohexyle, 2-methoxyethyle, 2-ethoxyethyle, 2-propoxyethyle, 2-butoxyethyle, 3-methoxypropyle, 3-ethoxypropyle, 3-propoxypropyle, 3-butoxypropyle, allyle, 2-buten-1-yle, 2-penten-1-yle, 2-octen-1-yle, benzyle, 4-methylbenzyle, alpha-methylbenzyle, alpha-ethylbenzyle, 2-, 3- et 4-fluorobenzyle, 2-, 3- et 4-chlorobenzyle, 4-bromobenzyle, 3- et 4-trifluoromethylbenzyle, 4-tert.-butylbenzyle, p-methoxybenzyle, 2,4-dichlorobenzyle ou phenylethyle et R représente, en outre, phenyle, 2-, 3- et 4-chlorophenyle, 4-methylphenyle, 4-methoxyphenyle, 4-trifluoromethyle, 2,4-dichloro et 3,4-dichlorophenyle, phenoxy-ethyle, -propyle, butyle et hexyle, 4-fluorophenoxyethyle et -butyle, 2-, 3- et 4-chlorophenoxy-ethyle, -propyle, -butyle, et hexyle, 2,4-, 3,4- et 2,6-dichlorophenoxy-ethyle, -propyle, -butyle et hexyle, 2,4,6-, 3,5,6- et 2,5,6-trichlorophenoxy-ethyle, -propyle et butyle, 4-methoxyphenoxyethyle, propyle et butyle, 2-methyl-4,6-dichlorophenoxyethyle, -propyle et -butyle, 2,6-dichloro-4-bromphenoxy-ethyle, -propyle et butyle, 2,6-dichloro-4-bromphenoxy-ethyle, -propyle et -butyle, 4-phenylphenoxy-ethyle, -propyle et -butyle et $R^2$ et $R^3$ représentent hydrogène, methyle ou isopropyle.

8. N-(azolylcarbamoyl)-hydroxylamine de formule I selon la revendication 1, caractérisé par le fait que R représente 4-fluorophenoxy-butyle, 2,4,6-trichlorophenoxy-propyle, 2,6-dichloro-4-bromo-phenoxy-ethyle, 4,6-dichloro-2-methyl-phenoxy-ethyle, $R^1$ représente ethyle ou allyle, Y, CH et $R^2$ et $R^3$ représentent hydrogène.